Europäisches Patentamt

European Patent Office

Office européen des brevets

(11) Publication number: 0 306 303
A1

# EUROPEAN PATENT APPLICATION

(21) Application number: 88308086.3

(22) Date of filing: 01.09.88

(51) Int. Cl.⁴: **A 61 K 39/40**
C 12 P 21/00

(30) Priority: 02.09.87 US 92070

(43) Date of publication of application:
08.03.89 Bulletin 89/10

(84) Designated Contracting States:
AT BE CH DE ES FR GB GR IT LI LU NL SE

(71) Applicant: SYNBIOTICS CORPORATION
11011 Via Frontera
San Diego California 92127 (US)

(72) Inventor: Vodian, Morton A.
1770 Sally Place
Escondido California 92026 (US)

Maggio, Edward T.
18775 Bernardo Trails Drive
San Diego California 92128 (US)

(74) Representative: Harrison, David Christopher et al
MEWBURN ELLIS & CO 2/3 Cursitor Street
London EC4A 1BQ (GB)

Claims for the following Contracting States: ES + GR.

(54) Anti-viral neoantigen antibodies and anti-idiotypic antibodies induced thereby as anti-viral agents.

(57) Novel anti-viral agents comprising antibodies that bind to viral neoantigens are provided. Anti-idiotype antibodies induced thereby and anti-drug receptor antibodies are also provided.

**Description**

# ANTI-VIRAL NEOANTIGEN ANTIBODIES AND ANTI-IDIOTYPIC ANTIBODIES INDUCED THEREBY AS ANTI-VIRAL AGENTS

The present invention relates to anti-viral agents and in particular to anti-viral neoantigen antibodies and anti-idiotypic antibodies induced thereby.

Many anti-viral drugs which are highly effective in tissue culture cannot be used in patients because of adverse side effects. In particular, many anti-viral agents which are found to be effective in culture have side effects ranging from vomiting, insomnia, and dizziness to severe cytotoxicity when administered to patients. Anti-viral agents with minimal side effects in the host animal that still induce effective virus inactivation are desirable.

In providing protection from viruses, we are limited by the fact that agents effective against bacteria are not usually useful against viral invasion because viruses utilize endogenous cellular machinery for their proliferation and viruses spend a portion of their existence within host cells. Being able to inhibit virus-induced diseases without significant harm to the host is a daunting task.

## RELEVANT LITERATURE

It has been demonstrated that certain biologically active protein molecules undergo conformational changes during the course of expression of their activity. The conformational changes reveal new antigenic epitopes on the molecules which are referred to as neo-antigens. For example, the β-galactosidase tetramer, and G-proteins, undergo such conformational changes. In another example, a component of the complement system binds to already established immune complexes. That interaction exposes complement binding sites on the Fc region of bound antibody. Thus, complement binds to a neoantigen epitope of the Fc region of antibody.

Smith et al., Science (1986) 223:1286-1293 described the three-dimensional structures of the drug binding site of rhinoviruses and related picornaviruses. Structurally related anti-viral agents were reported to inhibit picornavirus replication by preventing viral uncoating without affecting cellular attachment and penetration. Those compounds are described as stabilizing the virion against alkaline and heat denaturation. Smith et al. hypothesize that when the anti-viral compounds bind to the virus they interfere with the uncoating process.

Doms et al., J. Biol. Chem. (1985) 260:2973-2891 describe a pH-induced conformational change that influenza viruses undergo within the infected cell. After influenza virus binds to the surface of a susceptible cell, viral particles are endocytosed and transported to endosomes and lysosomes. The acidic environment of the organelles produces a pH-induced conformational change in the viral membrane and the viral membrane fuses with the cell membrane. The authors induced monoclonal antibodies that reacted with only one of the two conformations. Use of the antibodies reportedly indicated that of four epitopes present on HA1, two are lost when the virus undergoes the conformational change and at least one new epitope is exposed.

A variety of anti-viral agents are known to bind covalently or non-covalently to virus, stabilizing a non-infectious conformation. McKinlay et al., Antimicrob. Agents and Chemother. (1982) 22:1022-1025 report that arildone stabilizes Semliki Forest virus, vesicular stomatitis virus, coxsackievirus A9, murine cytomegalovirus, herpes simplex virus types I and II, and poliovirus to alkaline and thermal degradation. The stabilization is reported to affect uncoating, inhibiting plaque formation. The authors determined that use of the drug was effective in preventing polio-virus-induced paralysis and death in mice. Superti et al., J. Gen. Virol. (1984) 65:781 reported that ammonium chloride and chloroquine prevented rabies virus fusion with susceptible cells, preventing virus infection.

Binding by anti-viral agents to viral protein receptor sites can be increased or made covalent by altering the structure of the anti-viral agent. For example, Diana et al., J. Med. Chem. (1985) 28:1906-1910 report that [[(4,5-dihydro-2-oxazolyl)phenoxy]alkyl]isoxazoles are antiviral agents that prevent uncoating of picornaviruses, rhinoviruses and enteroviruses. Those authors reported the differences in anti-viral activity that correlated with chain length. 5-[[4-(4,5-dihydro-2-oxazolyl)phenoxy)alkyl]-3-methylisoxazoles reportedly binds to picornaviruses. inhibiting viral uncoating. Diana et al., J. Med. Chem. (1987) 30:383, reported that substituents in the 2-position greatly enhanced anti-viral activity.

## SUMMARY OF THE INVENTION

The present invention concerns novel anti-viral agents comprising antibodies or fragments thereof that specifically bind to a viral neoantigen. A viral neoantigen is a protein having a conformation-dependent epitope which is expressed when the virus is non-infectious but not when the virus is infectious.

Anti-idiotype antibodies useful as immunogens for inducing anti-viral neoantigen antibodies are also provided.

Methods of producing and using anti-viral neoantigen antibodies and anti-idiotype antibodies induced thereby are also provided.

## DESCRIPTION OF SPECIFIC EMBODIMENTS

Definitions

NATIVE CONFORMATION - the predominant conformation of a viral protein when the virus is in its native state and the conformation of the viral protein when the virus is capable of infecting a susceptible cell.

VIRAL NEOANTIGEN - a viral protein having a conformation-dependent epitope which is exposed on the protein when the virus has substantially reduced ability to infect a susceptible cell or is non-infectious and absent or inaccessible when the virus is infectious. Viral neoantigens comprise one or more conformation-dependent epitopes on a viral protein which can be induced by binding an anti-viral drug to the protein.

ANTI-(VIRAL NEOANTIGEN) ANTIBODY - an antibody specific for a viral neoantigen epitope.

DRUG RECEPTOR SITE - a site on a virus to which an anti-viral drug binds resulting in substantially diminished infectivity of the virus due to a change in conformation resulting in formation of neoantigen.

The present invention provides novel anti-viral agents comprising antibodies or fragments of antibodies that bind to a viral neoantigen epitope resulting in loss of infectivity. Anti-idiotype antibodies useful as immunogens for inducing anti-viral neoantigen antibodies in a vertebrate host are also provided.

The interaction of anti-viral agents with viruses creates three-dimensional changes in the virus. The anti-viral agent-induced changes create neoantigens of the viral proteins. Normally there will be an equilibrium between the predominant three-dimensional conformation assumed by viral surface proteins when the virus is infectious (the native conformation) and the three-dimensional conformation which exhibits viral neoantigens (NEO). Anti-viral agent-induced conformational changes in the virus protein drive the equilibrium in the direction of the conformation exhibiting NEO. Those conformational changes can be stabilized and maintained by the use of antibodies which bind with viral neoantigen epitopes.

Since a virus is inactivated when the viral proteins are in a conformation that expresses neoantigens, driving the virus into or retaining the virus in that conformation is one way to inactivate virus. Anti-viral neoantigen (anti-NEO) antibodies are thus anti-viral agents.

To prepare anti-NEO antibodies, the NEO conformation must be induced to serve as an immunogen. While the use of covalently bound drugs is preferred for the induction of NEO, drugs which bind non-covalently with viral proteins with high affinity ($>10^8 M^{-1}$) are also suitable. A number of such drugs are described in the Background section. Usually, one or more anti-viral drugs which induce NEO are mixed with intact virus and the resulting drug-conjugated virus is used in a suitable diluent as an immunogen. Alternatively, the immunogen may be a viral protein, rather than intact virus.

The viral protein used is in a conformation of the viral protein as part of the virus in a non-infectious conformation. Conveniently, the viral protein contains the ligand site for binding to a susceptible host cell membrane protein. The viral protein used may be a substantially intact virus capsid protein, envelope protein, or a portion of a virus protein, so long as the protein is in the non-infectious conformation. The virus protein-drug conjugate can be bound to a carrier to make the conjugate immunogenic if the conjugate is not immunogenic per se. Carriers include bovine serum albumin, keyhole limpet hemocyanin and the like.

The immunogen comprises the virus protein-drug conjugate in a suitable diluent. The diluent disperses the conjugate and can be used to enhance the immunogenicity if desired, as where the viral protein-drug conjugate is not sufficiently immunogenic per se. Suitable diluents are water, saline, buffered salines, complete or incomplete adjuvants and the like. The immunogen is administered to the host animal using standard techniques for antibody induction.

The host animal's serum or plasma may be collected following an appropriate time interval to provide a composition of anti-NEO antibodies. The gamma globulin fraction or the IgG antibodies can be obtained by use of saturated ammonium sufate or DEAE Sephadex, respectively, as is known. To enhance the specificity of the isolated anti-NEO antibody composition, the composition can be purified by adsorbing the preparation with infectious virus. That adsorption removes antibodies in the preparation that are specific for viral epitopes which are not neoantigen epitopes. The composition can also be adsorbed against the drug to remove any drug-specific antibodies that may be present in the composition.

Anti-NEO antibodies are useful as anti-viral agents. Upon administration to an infected host animal, the antibody binds to and stabilizes virus in the non-infectious NEO conformation. In addition, anti-NEO antibodies also find use as immunogens to induce anti-idiotype antibody.

The anti-idiotype antibody so produced is specific for the binding site region of anti-NEO antibodies and thus mimics the neoantigen epitope for which the inducing antibody was specific. The anti-idiotype antibody will be referred to as "mimic antibody" since the binding region mimics a specific epitope to which the anti-neoantigen antibody binds. Conveniently, a purified anti-NEO antibody preparation is used to induce mimic antibody in a host animal. The composition is administered to the host animal in a suitable diluent. Following administration, usually repeated administration, the host produces mimic antibody. To eliminate an immunogenic response to the Fc region, antibodies produced by the same species as the host animal can be used or the Fc region of the administered antibodies can be removed.

Following induction of mimic antibody in the host, serum or plasma is removed to provide an antibody composition. The composition can be purified to obtain the gamma-globulin or IgG fraction to enhance the antibody concentration. The composition can also be purified by binding the composition to immobilized anti-NEO antibodies to enhance the specificity of the composition. Mimic antibodies remain bound to immobilized anti-NEO antibodies while antibodies of other specificity can be removed. Thereafter, bound mimic antibodies can be released to provide a purified composition of mimic antibodies.

Alternatively, monoclonal anti-NEO antibodies or monoclonal mimic antibodies can be produced. The spleen or lymphocytes from an immunized animal are removed and immortalized. Hybridomas can be produced by following Kohler and Milstein's method.

To produce a human-human hybridoma, a human lymphocyte donor is selected. A patient known to have a viral infection (e.g., where infection has been shown by either viral serological assays or virus culture) may serve as a suitable lymphocyte donor. The patient's lymphocytes are removed. Peripheral blood lymphocytes can be isolated from a relatively small blood sample. Alternatively, spleen cells can be used if the patient is subject to splenectomy. Epstein-Barr virus (EBV) can be used to immortalize human lymphocytes or a human fusion partner can be used to produce human-human hybridomas.

Antibodies secreted by the immortalized cells are screened to determine the clones that secrete antibodies of the desired specificity. For monoclonal anti-NEO antibodies, the antibodies must bind to virus in the NEO conformation, but not to virus in the native conformation or the drug used to induce NEO, but recognizing that binding will drive native virus into the NEO conformation. Thus, the rate of antigen binding to virus will be substantially smaller than the rate of binding to drug-virus complex. For monoclonal mimic antibodies, the antibodies must bind to the binding site region of anti-NEO antigen antibodies. Cells producing antibodies of the desired specificity would be selected.

Anti-NEO antibodies are free of many of the adverse side effects of other anti-viral agents, since the materials are immunoglobulins. These materials can be made even more compatible with the host system by minimizing potential adverse immune system responses. This is accomplished by removing all or a portion of the Fc portion of a foreign species antibody or using an antibody of the same species as the host animal. For example, anti-NEO antibodies from human/ human hybridomas are suitable for treatment of patients.

Additionally, anti-NEO antibodies are useful in conjunction with other anti-viral drug therapy to enhance the effectiveness of the anti-viral drug. Anti-NEO antibodies stabilize a drug-induced conformation where the drug does not bind covalently; and they further shift the equilibrium to the inactive NEO conformation when used in conjunction with a covalently bound drug. That action increases the percentage of the virus which is present in the inactive, non-infectious conformation. This enhancement may allow the drug to be administered in lower dosages, minimizing adverse side effects of the drug.

The use of an effective amount of anti-NEO antibodies can also enhance the host animal's immune response since antibody-virus complexes are recognized by macrophages. Anti-NEO antibodies can be administered in amounts similar to those used for other therapeutic administration of antibody. For example, pooled gamma globulin is administered at 0.02-0.1 ml/lb body weight during the early incubation of rabies, measles, hepatitis, polio and other viral diseases to interfere with viral entry into cells. Thus, anti-NEO antibodies can be passively administered alone or in conjunction with another anti-viral agent to a virus-infected host animal to enhance the animal's immune response and the effectiveness of the antiviral drug.

Alternatively, mimic antibody can be administered to the host animal to induce anti-NEO antibodies in the host. The manner of injecting the antibody is the same as for vaccination purposes. That is, the antibody can be injected intramuscularly, intraperitoneally, subcutaneously or the like in an effective concentration in a physiologically suitable diluent with or without adjuvant. One or more booster injections may be desirable. This induction of anti-NEO antibodies would alleviate problems which might be caused by passive administration of anti-NEO antibodies. Potential problems such as an adverse immune response, particularly if the antibodies are of another species, and problems associated with administration of blood products, such as infection, would be eliminated.

An additional advantage of the invention is that many anti-viral drugs which are highly cytotoxic in the virus natural host can be tested for effectiveness in tissue culture. Drugs which produce their anti-viral effects by inhibiting attachment, penetration or uncoating may produce conformational changes in a viral protein, inducing NEO. Those drugs which prove to be highly effective in tissue culture but cannot be used on patients because of adverse side effects can be used to induce neoantigen. Anti-NEO antibodies can be generated in an appropriate host animal against the drug-induced conformation. In this way, anti-NEO antibodies can be used as a therapeutic agent to stabilize the same viral protein conformation as induced by those cytotoxic agents. Thus, anti-NEO antibodies can be passively administered to the host or induced in the host by mimic antibody to stabilize the same conformation as would be induced by use of the most effective anti-viral drugs.

A further use of anti-NEO antibodies is in the rational design of anti-viral agents. Anti-viral agents which exhibit the maximum ability to inhibit a specific virus in tissue culture may be selected. Anti-NEO antibodies can be induced by immunizing with intact or substantially intact virus bound to an effective compound. Upon mixing anti-NEO antibodies with infectious virus, the inactive viral protein conformations which possess neoantigens can now be stabilized in the absence of the drug which originally initiated the NEO conformation. Thus, the drug receptor site can be exposed while the viral protein is maintained in the inactive conformation. Polyclonal or monoclonal anti-drug receptor antibodies can be generated to a variety of epitopes in and around the drug receptor site. Those anti-drug receptor antibodies can be tested to determine which anti-receptor antibodies mimic the function of the drug in inducing NEO. A portion of the antibodies may exhibit anti-viral activity which is related in function to the anti-viral agent which originally induced the NEO conformation. Thus, an immunoglobulin or fragment thereof which mimics the function of the anti-viral drug is produced.

Further, the antibodies produced herein may be enzymatically digested to provide smaller fragments which still exhibit anti-viral activity. In particular, Fab, $F(ab')_2$ and Fab' portions of antibodies retain the binding ability of intact antibody and do not contain the Fc portion which would be likely to be immunogenic if produced by a different species of animal than the host animal. Additionally, these smaller fragments which retain their anti-viral function may thereafter be sequenced and produced synthetically or by recombinant DNA techniques. Also, genetically engineered Fv regions and chimeras which have mouse variable and human constant regions have been produced. (Morrison et al., Proc. Natl. Acad. Sci. USA (1984) 81:6851-6855.)

When an animal having anti-neoantigen antibodies is exposed to a virus, that portion of the viral population exhibiting neoantigens is stabilized with the viral protein in the inactive conformation. The equilibrium is driven toward NEO and, thus, inactivated virus. The equilibrium can be further driven toward NEO by the use of anti-viral drugs in conjunction with the anti-NEO antibodies. Anti-NEO antibodies can be either induced in the host by prior immunization with mimic antibodies or can be passively administered to the host animal during viral infection. Additionally, anti-drug receptor site antibodies can be administered to an infected animal and enhance virus inactivation and the body's immune response to the virus.

The following examples are provided by way of illustration and not by way of limitation.

EXPERIMENTAL

Example 1 - Production of Monoclonal Anti-NEO Antibodies

Arildone (Sterling Drug, Inc. New York) is added to a purified preparation of poliovirus (1.0 μM arildone per $10^3$ pfu poliovirus - $10^3$ pfu poliovirus is 1 $LD_{50}$ in Balb/c mice) and allowed to stand on ice for two hours to allow arildone to bind to the virus. The virus-drug mixture is mixed with complete Freund's adjuvant (CFA) prior to use to provide 20 μg of virus-arildone per 0.1 ml conjugate mixture. Three female Balb/c mice seven weeks old are injected intraperitoneally with 20 μg per mouse of poliovirus-arildone in adjuvant. Twenty eight days later each mouse is boosted with an intravenous injection of 10 μg of poliovirus-arildone in phosphate buffered saline. Four days after the boost, spleen cells are removed and fused to P3-X63/Ag8/653, a non-secreting myeloma cell line using 50% polyethylene glycol 1540 following the Kohler and Milstein procedure. Fused cells are selected in 96 well tissue culture plates using HAT medium and limiting dilution.

Monoclonal antibodies produced by each hybridoma cell clone are assayed to determine their specificity. A hybridoma which produces monoclonal antibodies that react in an ELISA assay with poliovirus bound to arildone but reacts more weakly to poliovirus or to arildone preparations is selected. This hybridoma is then used in a competitive inhibition assay to determine the cross-reactivity of the neoantigen epitope to which it binds.

Example 2 - Production of Monoclonal Mimic Antibody

Balb/c mice are used to produce monoclonal mimic antibody. The mice are immunized using 20 μg each of purified monoclonal antibody produced in Example 1. Fusion is performed in the same manner. Hybridoma cells which produce monoclonal antibodies that bind to purified monoclonal anti-NEO antibodies are selected and retained.

Example 3 - Use of Mimic Antibodies to Induce Anti-NEO Antibodies

Purified anti-neoantigen antibodies produced by a Balb/c mouse in Example 2 are injected into three Balb/c mice. A first injection of 50 μl of purified antibody in CFA is followed by two injections of 50 μl in incomplete Freunds adjuvant (IFA) at four week intervals. The mice are bled to determine anti-NEO antibody titer two weeks after the last injection. One of the mice is determined to have a lower antibody titer than the others and is given a booster of 50 μl of purified antibody.

All three mice are bled and the collected serum is pooled. The pooled serum is assayed by ELISA as in Example 1 to determine antibody specificity.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced.

**Claims**

1. An anti-viral agent comprising an antibody composition, said composition comprising antibodies specific for a viral neoantigen, said neoantigen being a conformation-dependent epitope which is present on a virus protein when said virus has substantially reduced ability to infect a susceptible cell and absent, or diminished when said virus is infectious.

2. An antibody composition according to claim 1 wherein said antibody binds to a virus-antiviral drug complex without binding to said drug and is substantially free of binding to said virus when said virus is not bound to said anti-viral drug.

3. An antibody composition produced by administering to a host animal an immunogenic composition comprising in a suitable diluent a viral protein bound to an anti-viral drug wherein said viral protein is in a conformation of said viral protein, provided that when said viral protein is part of said virus, said virus is in a non-infectious conformation.

4. The antibody composition according to claim 1 wherein said viral protein comprises a ligand site for binding to a host cell membrane protein resulting in infection of said host cell.

5. The antibody composition according to claim 3 or claim 4 wherein said protein is administered as a component of a substantially intact virus.

6. The anti-viral agent according to any of the preceding claims wherein said antibody composition is monoclonal.

7. An anti-idiotype antibody composition specific to an anti-viral neoantigen antibody that competitively inhibits binding of said anti-viral neoantigen antibody to a viral neoantigen.

8. An active fragment of an antibody composition of any of claims 1, 2, 3, 8 or 11, comprising a polypeptide comprising the binding site portion of said antibody.

**Claims for the following Contracting States:ES,GR**

1. A method of preparing an anti-viral agent which includes preparing an antibody composition, said composition comprising antibodies specific for a viral neoantigen, said neoantigen being a conformation-dependent epitope which is present on a virus protein when said virus has substantially reduced ability to infect a susceptible cell and absent, or diminished when said virus is infectious.

2. A method according to claim 1 wherein said antibody binds to a virus-antiviral drug complex without binding to said drug and is substantially free of binding to said virus when said virus is not bound to said anti-viral drug.

3. A method of preparing an antibody composition by administering to a host animal an immunogenic composition comprising in a suitable diluent a viral protein bound to an antiviral drug wherein said viral protein is in a conformation of said viral protein, provided that when said viral protein is part of said virus, said virus is in a non-infectious conformation.

4. A method according to claim 3 wherein said viral protein comprises a ligand site for binding to a host cell membrane protein resulting in infection of said host cell.

5. A method according to claim 3 or claim 4 wherein said protien is administered as a component of a substantially intact virus.

6. A method according to any of the preceding claims wherein said antibody composition is monoclonal.

7. A method of preparing an anti-idiotype antibody composition specific for an anti-viral neoantigen antibody that competitively inhibits binding of said antiviral neoantigen antibody to a viral neoantigen.

8. A method according to any one of the preceding claims including using an active fragment of the antibody composition, the fragment comprising a polypeptide comprising the binding site portion of said antibody.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF IMMUNOLOGY, vol. 134, no. 2, February 1985, pages 1225-1229, The American Association of Immunologists, US; F.G.C.M. UYTDEHAAG et al.: "Induction of neutralizing antibody in mice against poliovirus type II with monoclonal anti-idiotypic antibody" * Whole article * | 1-8 | A 61 K 39/40<br>C 12 P 21/00 |
| X | WO-A-8 303 972 (MASSACHUSETTS INSTITUTE OF TECHNOLOGY) * Claims * | 1-6 | |
| A | EP-A-0 112 741 (INSTITUT PASTEUR) | | |

TECHNICAL FIELDS SEARCHED (Int. Cl.4)

A 61 K
C 12 P

The present search report has been drawn up for all claims

| Place of search | Date of completion of the search | Examiner |
|---|---|---|
| THE HAGUE | 28-10-1988 | TURMO Y BLANCO C.E. |